# EUROPEAN PATENT APPLICATION

(11) **EP 4 410 948 A1**
(43) Date of publication of application: **07.08.2024**
(21) Application number: 23182002.8
(22) Date of filing: 28.06.2023
(51) Int. Cl.: C12M 1/32, C12M 3/06, C12M 1/12, C12M 1/42, C12M 1/00

(54) **THREE-DIMENSIONAL CULTURE DEVICE AND BIOLOGICAL CULTURE INSTRUMENT**

(30) Priority: 02.02.2023 US 202363442776 P
(71) Applicant: Transformative Cell Processing Co., Ltd., Taipei City, 10563 (TW)
(72) Inventor: HUANG, Chung-Er, 30261 Zhubei City, Hsinchu County (TW); KUO, Hsu-Sung, 10563 Taipei City (TW); CHANG, Mau-Chung Frank, 10563 Taipei City (TW)
(74) Representative: Viering, Jentschura & Partner mbB Patent- und Rechtsanwälte

(57) **Abstract**

A three-dimensional culture device (100) and a biological culture instrument (100a) are provided. The biological culture instrument (100a) is configured to receive a culture medium (3) and includes a container (1), a cover (2), and a driving mechanism (4) assembled to the cover (2). The container (1) includes a plurality of accommodating wells (13) each having a filtering bottom structure (111) and a surrounding wall (121) connected to the filtering bottom structure (111). Each of the surrounding walls (121) defines a culture space (S) for accommodating the culture medium (3) and at least one bioparticle (P). The cover (2) includes a communication portion (21) and a plurality of insertion pipes (22) being in spatial communication with the communication portion (21). Each of the insertion pipes (22) is inserted into one of the accommodating wells (13), and is in spatial communication with the culture space (S) through the filtering bottom structure (111). The driving mechanism (4) is configured to control volume of the culture medium (3) in each of the culture spaces (S) through the cover (2).

## Description

### FIELD OF THE INVENTION

The present invention relates to a culture instrument, and more particularly to a three-dimensional culture device and a biological culture instrument.

### BACKGROUND OF THE INVENTION

A conventional biological culture instrument is provided to accommodate a culture medium through each of chambers thereof, so that bioparticles can be cultured through the culture medium in each of the chambers. Moreover, a volume of the culture medium in each of the chambers is controlled through regulation of air pressure. However, due to the compressibility of air, controlling the volume of the culture medium in this manner can be unstable, and may not be suitable for culturing of the bioparticles.

### SUMMARY OF THE INVENTION

In response to the above-referenced technical inadequacies, the present invention provides a three-dimensional culture device and a biological culture instrument to effectively improve on the issues associated with conventional biological culture instruments.

In order to solve the above-mentioned problems, one of the technical aspects adopted by the present invention is to provide a three-dimensional (3D) culture device for culturing a plurality of bioparticles. The 3D culture device includes a plurality of accommodating wells, a cover, a culture medium, and a driving mechanism. Each of the accommodating wells has a filtering bottom structure and a surrounding wall that is connected to the filtering bottom structure. The surrounding wall of each of the accommodating wells defines a culture space therein for accommodating at least one of the bioparticles. The cover includes a communication portion and a plurality of insertion pipes that are connected to the communication portion. The insertion pipes are in spatial communication with each other through the communication portion. The insertion pipes are respectively inserted into the culture spaces of the accommodating wells, and each of the insertion pipes is in spatial communication with the corresponding culture space through the filtering bottom structure of the corresponding accommodating well. The culture medium is filled in the culture spaces of the accommodating wells, the communication portion of the cover, and the insertion pipes of the cover, so that the bioparticles respectively located in the culture spaces are immersed in the culture medium. The driving mechanism is assembled to the cover. The driving mechanism is configured to drive the culture medium in the cover so as to allow the culture medium to flow into or out of each of the culture spaces for controlling the volume of the culture medium in each of the culture spaces.

In order to solve the above-mentioned problems, another one of the technical aspects adopted by the present invention is to provide a biological culture instrument for accommodating a culture medium to allow a plurality of bioparticles to be cultured therein. The biological culture instrument includes a container, a cover, and a driving mechanism. The container includes a plurality of accommodating wells spaced apart from each other. Each of the accommodating wells has a filtering bottom structure and a surrounding wall that is connected to the filtering bottom structure. The surrounding wall of each of the accommodating wells defines a culture space therein for accommodating the culture medium and at least one of the bioparticles. The cover includes a communication portion and a plurality of insertion pipes that are connected to the communication portion. The insertion pipes are in spatial communication with each other through the communication portion. The insertion pipes are respectively inserted into the culture spaces of the accommodating wells, and each of the insertion pipes is in spatial communication with the corresponding culture space through the filtering bottom structure of the corresponding accommodating well. The driving mechanism is assembled to the cover. The driving mechanism is configured to control the volume of the culture medium in each of the culture spaces through the cover.

In order to solve the above-mentioned problems, yet another one of the technical aspects adopted by the present invention is to provide a three-dimensional (3D) culture device for culturing a plurality of bioparticles. The 3D culture device includes a container, a cover, a culture medium, and a driving mechanism. The container includes a plurality of accommodating wells spaced apart from each other. Each of the accommodating wells has a bottom structure and a surrounding wall that is connected to the bottom structure. The surrounding wall of each of the accommodating wells defines a culture space therein for accommodating at least one of the bioparticles. The cover includes a communication portion and a plurality of insertion pipes that are connected to the communication portion. The insertion pipes are in spatial communication with each other through the communication portion. Each of the insertion pipes has a plurality of filtering holes, the insertion pipes are respectively inserted into the culture spaces of the accommodating wells, and each of the insertion pipes is in spatial communication with the corresponding culture space through the filtering holes thereof. The culture medium is filled in the culture spaces of the accommodating wells, so that the bioparticles respectively located in the culture spaces are immersed in the culture medium. The driving mechanism is assembled to the cover. The driving mechanism is configured to drive the culture medium in the cover so as to allow the culture medium to flow into or out of each of the culture spaces for controlling the volume of the culture medium in each of the culture spaces.

Therefore, in any one of the 3D culture device and the biological culture instrument provided by the present embodiment, the structural configuration of the accommodating wells and the cover (e.g., the culture spaces being in spatial communication with the insertion pipes and the communication portion for facilitating filling of the culture medium) enables the volume of the culture medium in each of the accommodating wells to be stably controlled in a liquid-pressure manner, thereby facilitating the culturing of the bioparticles.

These and other aspects of the present invention will become apparent from the following description of the embodiment taken in conjunction with the following drawings and their captions, although variations and modifications therein may be affected without departing from the scope of the novel concepts of the invention.

### BRIEF DESCRIPTION OF THE DRAWINGS

The described embodiments may be better understood by reference to the following description and the accompanying drawings, in which:
FIG. 1 is a schematic perspective view of a three-dimensional (3D) culture device according to a first embodiment of the present invention;
FIG. 2 is a schematic exploded view of FIG. 1 when a culture medium is omitted;
FIG. 3 is a schematic top view of FIG. 1;
FIG. 4 is a schematic cross-sectional view taken along line IV-IV of FIG. 3;
FIG. 5 is a schematic enlarged view of part V of FIG. 4;
FIG. 6 is a schematic cross-sectional view of the 3D culture device in another configuration according to the first embodiment of the present invention;
FIG. 7 is a schematic cross-sectional view showing a first operation of FIG. 4;
FIG. 8 is a schematic cross-sectional view showing a second operation of FIG. 4;
FIG. 9 is a schematic cross-sectional view of the 3D culture device according to a second embodiment of the present invention;
FIG. 10 is a schematic cross-sectional view of the 3D culture device according to a second embodiment of the present invention; and
FIG. 11 is a schematic enlarged view of part XI of FIG. 10.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

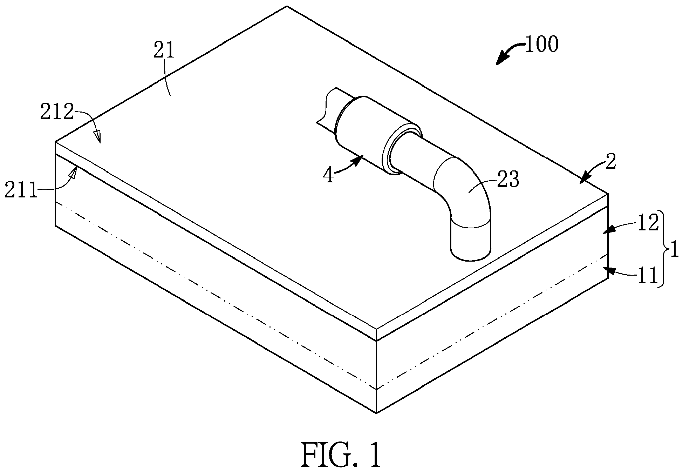

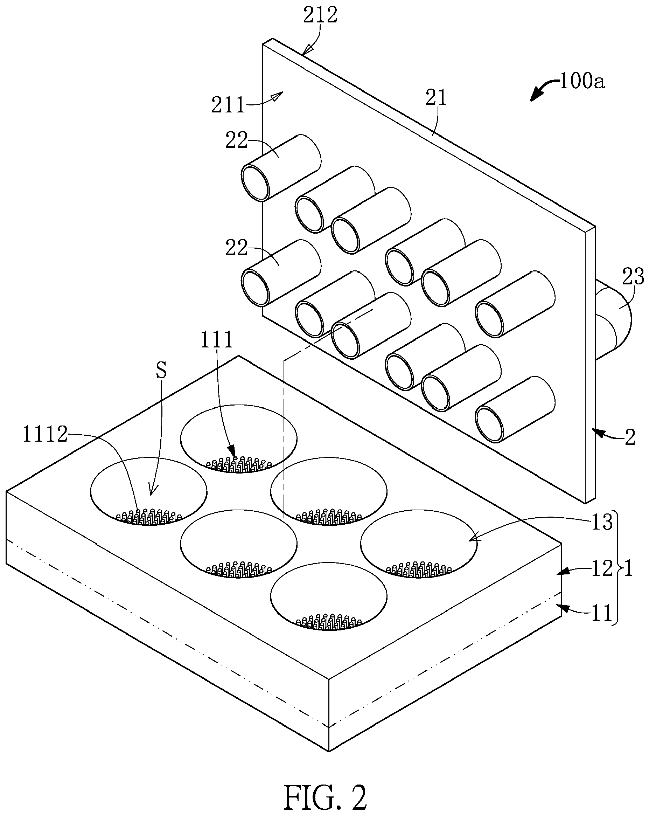

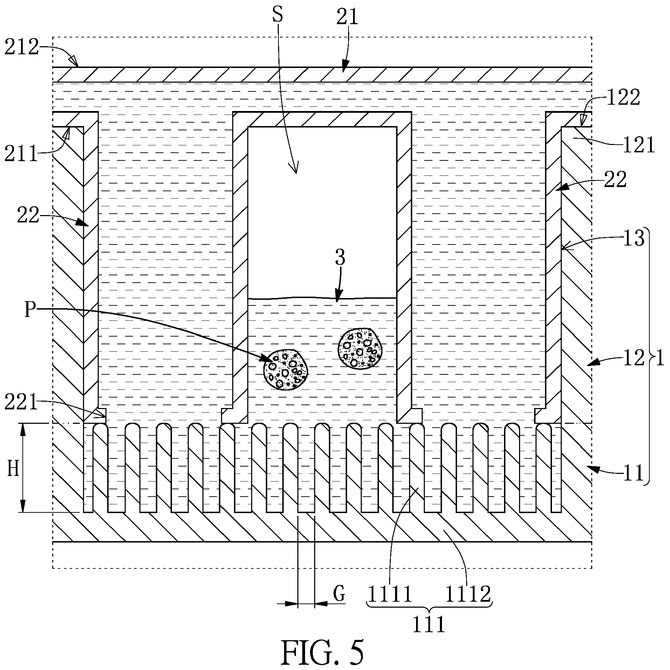

Referring to FIG. 1 to FIG. 8, a first embodiment of the present invention is provided. As shown in FIG. 1 to FIG. 4, the present embodiment provides a three-dimensional (3D) culture device 100 for culturing a plurality of bioparticles P. The 3D culture device 100 in the present embodiment includes a container 1, a cover 2 assembled to the container 1, a culture medium 3 (e.g., a nutrient solution) filled in the container 1 and the cover 2, and a driving mechanism 4 that is assembled to the cover 2, but the present invention is not limited thereto.

In other words, the container 1, the cover 2, and the driving mechanism 4 in the present embodiment can be jointly defined as a biological culture instrument 100a for accommodating the culture medium 3 to allow the bioparticles P to be cultured therein. It should be noted that the biological culture instrument 100a in the present embodiment is described in cooperation with the culture medium 3, but the present invention is not limited thereto. For example, in other embodiments of the present invention not shown in the drawings, the biological culture instrument 100a can be independently used (e.g., sold) or can be used in cooperation with other components.

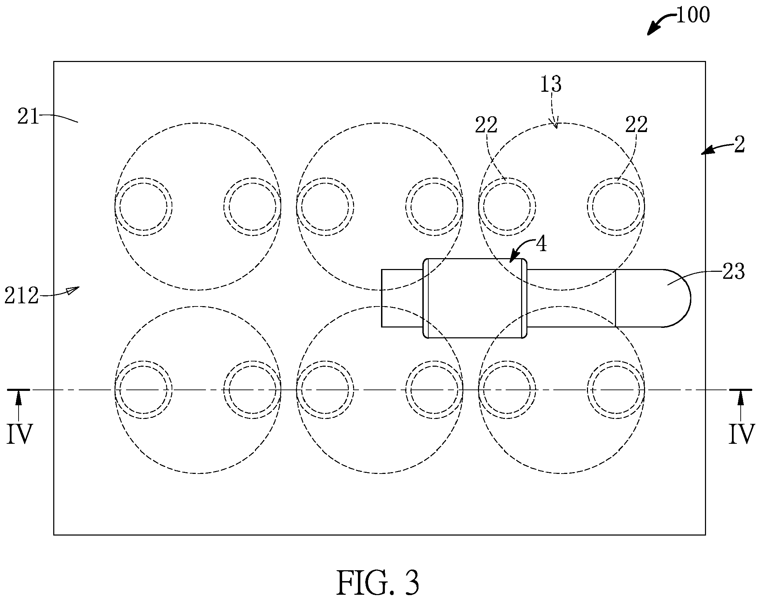

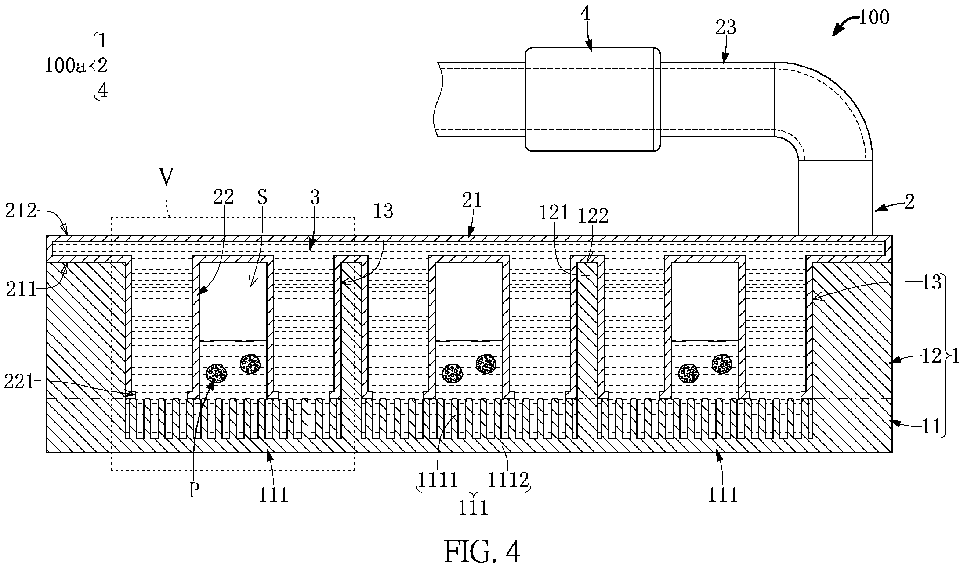

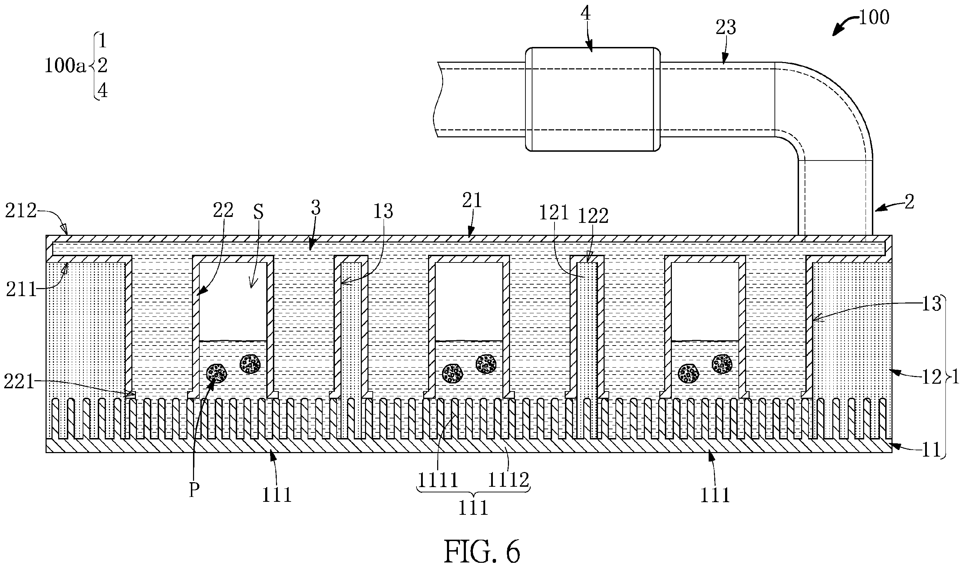

As shown in FIG. 3 to FIG. 5 of the present embodiment, the container 1 includes a carrier 11 and a frame 12 that is connected to the carrier 11. The carrier 11 and the frame 12 jointly form (or define) a plurality of accommodating wells 13 that are spaced apart from each other and that have a fixed relative position. It should be noted that the container 1 (e.g., the carrier 11 and the frame 12) can be a semiconductor chip or a substrate, thereby facilitating forming of a structure or a size that is suitable for culturing the bioparticles P. However, the specific structure or size of the container 1 can be changed or adjusted according to design requirements (e.g., as shown in FIG. 4, the carrier 11 and the frame 12 are integrally formed as a single one-piece structure; or, as shown in FIG. 6, the frame 12 can be additionally formed on the carrier 11), and the present invention is not limited thereto.

Specifically, the carrier 11 has a plate-like shape, and the carrier 11 has a plurality of filtering bottom structures 111 that are arranged on a top surface thereof and that are spaced apart from each other. In addition, the filtering bottom structures 111 have a fixed relative position. The frame 12 has a plurality of surrounding walls 121 spaced apart from each other and a top end surface 122 that is connected to the surrounding walls 121 and that is arranged away from the carrier 11. Each of the filtering bottom structures 111 and the corresponding surrounding wall 121 connected thereto are jointly defined as one of the accommodating wells 13. As the accommodating wells 13 in the present embodiment are of the substantially same structure, the following description discloses the structure of just one of the accommodating wells 13 for the sake of brevity, but the present invention is not limited thereto. For example, in other embodiments of the present invention not shown in the drawings, the accommodating wells 13 can be of different structures.

Specifically, the surrounding wall 121 of the accommodating well 13 defines a culture space S therein for accommodating at least one of the bioparticles P (and the culture medium 3). The filtering bottom structure 111 includes a base 1112 and a plurality of upright arms 1111 that are connected to the base 1112 and that are spaced apart from each other. Any two of the upright arms 1111 adjacent to each other have a gap G therebetween being smaller than a diameter of any one of the bioparticles P, but the present invention is not limited thereto. For example, in other embodiments of the present invention not shown in the drawings, the filtering bottom structure 111 can be formed with another filtering configuration other than the upright arms 1111.

In the present embodiment, the gap G is less than or equal to 2 µm, and the upright arms 1111 can be in a regular arrangement (e.g., a matrix arrangement) or an irregular arrangement (e.g., a random arrangement) according to design requirements. The upright arms 1111 formed on the base 1112 have a density preferably enabling the filtering bottom structure 111 to have the gaps G that cannot allow the bioparticles P to free move or pass therethrough.

Moreover, the upright arms 1111 have an average height H being within a range from 5 µm to 15 µm. In other words, the heights of any two of the upright arms 1111 may have a slight difference therebetween, so that free ends of the upright arms 1111 can jointly define a culture environment having a wavy terrain. Each of the upright arms 1111 preferably has an elastic property, and the free end of each of the upright arms 1111 is not sharp, so that any one of the upright arms 1111 can make a swinging motion when being in contact with the bioparticles P for avoiding any harming the bioparticles P, but the present invention is not limited thereto.

The cover 2 is formed to correspond in shape with the container 1, and the cover 2 includes a communication portion 21, a plurality of insertion pipes 22 connected to the communication portion 21, and an external pipe 23 that is connected to the communication portion 21. In the present embodiment, the communication portion 21 has a plate-like shape, and the communication portion 21 has a bottom side 211 and a top side 212 that is opposite to the bottom side 211. The insertion pipes 22 are connected to the bottom side 211 of the communication portion 21, and the external pipe 23 is connected to the top side 212 of the communication portion 21, but the present invention is not limited thereto. For example, in other embodiments of the present invention not shown in the drawings, the external pipe 23 of the cover 2 can be omitted or can be replaced by other components.

Moreover, the insertion pipes 22 are in spatial communication with each other through the communication portion 21, and the external pipe 23 is in spatial communication with each of the insertion pipes 22 through the communication portion 21. Specifically, the communication portion 21 in the present embodiment can be a hollow structure, but the present invention is not limited thereto. For example, in other embodiments of the present invention not shown in the drawings, the cover 2 can have a channel that is formed in the communication portion 21 for being in spatial communication with the insertion pipes 22 and the external pipe 23.

Specifically, the cover 2 is detachably assembled to the container 1, and the insertion pipes 22 are respectively inserted into the culture spaces S of the accommodating wells 13 (e.g., each of the accommodating wells 13 can accommodate at least one of the insertion pipes 22 therein), and each of the insertion pipes 22 is in spatial communication with the corresponding culture space S through the filtering bottom structure 111 of the corresponding accommodating well 13.

Moreover, the cover 2 in the present embodiment encloses an opening of each of the accommodating wells 13 that can be defined by the top end surface 122 (e.g., the communication portion 21 of the cover 2 abuts against the top end surface 122 to enclose the accommodating wells 13), so that the culture space S of each of the accommodating wells 13 is only in spatial communication with at least one of the insertion pipes 22 arranged therein. The cover 2 (or the insertion pipes 22) in the present embodiment can be in spatial communication with the culture spaces S only through the filtering bottom structures 111 of the container 1.

The culture medium 3 is filled in the culture spaces S of the accommodating wells 13 and the cover 2 (e.g., the communication portion 21, the insertion pipes 22, and the external pipe 23), so that the bioparticles P respectively located in the culture spaces S are immersed in the culture medium 3. In the present embodiment, since the accommodating wells 13 are enclosed by the cover 2, the air in each of the culture spaces S is maintained at a fixed amount.

Moreover, the culture medium 3 in the present embodiment can flow among the culture spaces S through the cover 2 and the filtering bottom structures 111 of the accommodating wells 13, so that the culture medium 3 is capable of transmitting biological information generated from the bioparticles P respectively located in the culture spaces S, thereby allowing a survival rate of the bioparticles P to be effectively increased.

The driving mechanism 4 in the present embodiment is a piezoelectric micro-controller, and the driving mechanism 4 is assembled to the external pipe 23 of the cover 2, but the present invention is not limited thereto. For example, in other embodiments of the present invention not shown in the drawings, the external pipe 23 of the cover 2 can be omitted, and the driving mechanism 4 is assembled to the communication portion 21 of the cover 2.

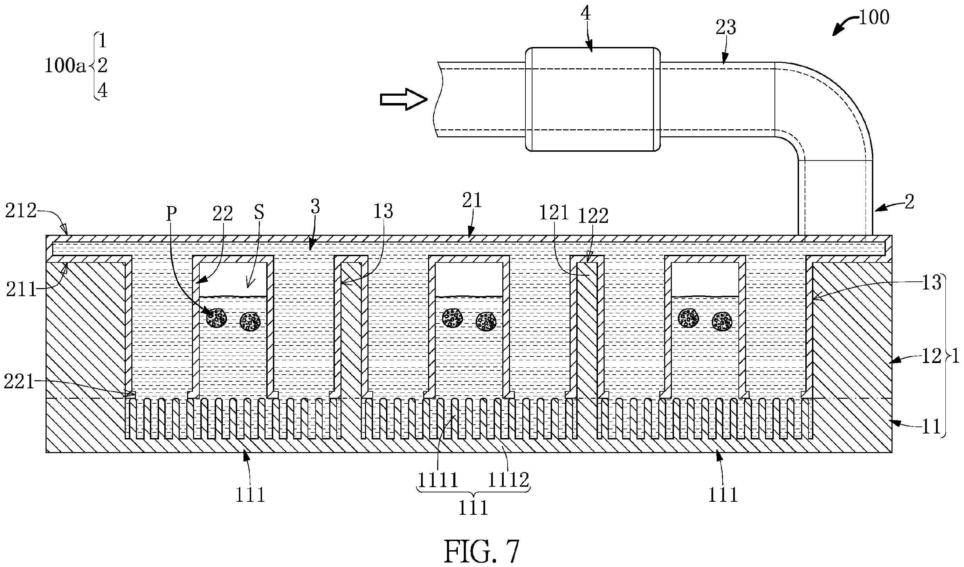

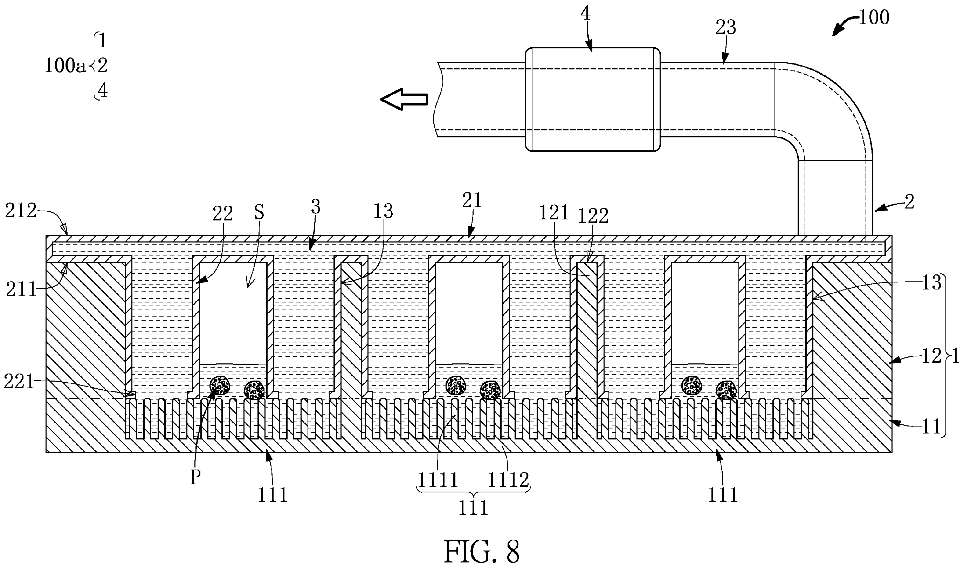

Accordingly, the driving mechanism 4 is provided for controlling volume of the culture medium 3 in each of the culture spaces S through the cover 2. Specifically, as shown in FIG. 7 and FIG. 8, the driving mechanism 4 is configured to drive the culture medium 3 in the cover 2 so as to allow the culture medium 3 to flow into or out of each of the culture spaces S for controlling the volume (or a liquid level) of the culture medium 2 in each of the culture spaces S. In the present embodiment, the 3D culture device 100 enables the liquid levels of the culture medium 3 in the culture spaces S to be the same, but the present invention is not limited thereto.

In summary, in the 3D culture device 100 (or the biological culture instrument 100a) provided by the present embodiment, the structural configuration of the accommodating wells 13 and the cover 2 (e.g., the culture spaces S being in spatial communication with the insertion pipes 22 and the communication portion 21 for facilitating filling of the culture medium 2) enables the volume of the culture medium 2 in each of the accommodating wells 13 to be stably controlled in a liquid-pressure manner, thereby facilitating the culturing of the bioparticles P.

### [Second Embodiment]

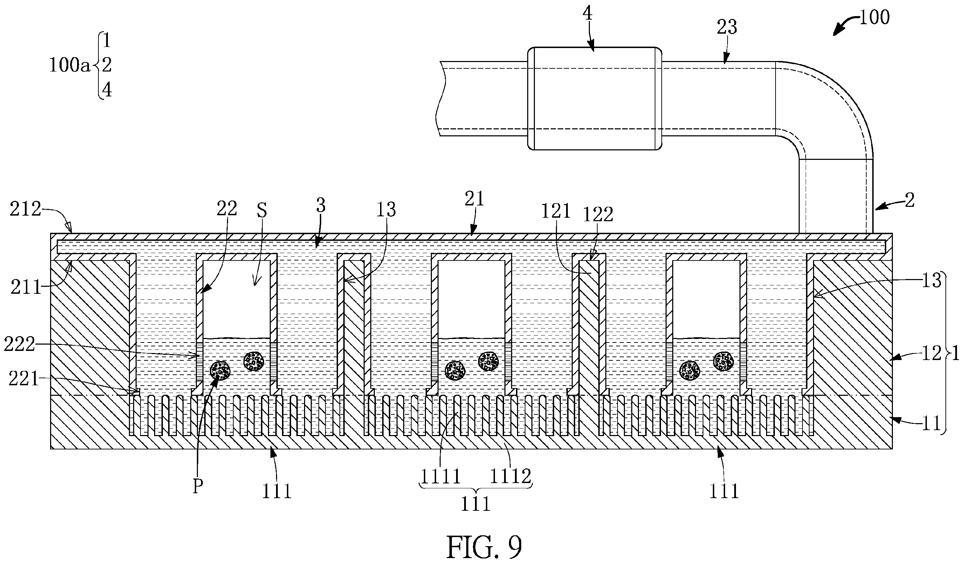

Referring to FIG. 9, a second embodiment of the present invention, which is similar to the first embodiment of the present invention, is provided. For the sake of brevity, descriptions of the same components in the first and second embodiments of the present invention will be omitted herein, and the following description only discloses different features between the first and second embodiments.

In the present embodiment, at least one of the insertion pipes 22 can have a plurality of filtering holes 222 formed on a portion thereof (e.g., a side wall) other than a nozzle 221 thereof. Moreover, the at least one of the insertion pipes 22 having the filtering holes 222 is able to be in spatial communication with the corresponding culture space S through the filtering holes 222 that are arranged adjacent to the corresponding filtering bottom structure 111 and that are immersed in the culture medium 3.

In other words, the cover 2 (or the insertion pipes 22) in the present embodiment can be in spatial communication with the culture spaces S through the filtering bottom structures 111 and the filtering holes 222. Furthermore, the filtering holes 222 of the at least one of the insertion pipes 22 preferably have an average aperture being less than 1 µm, but the present invention is not limited thereto.

### [Third Embodiment]

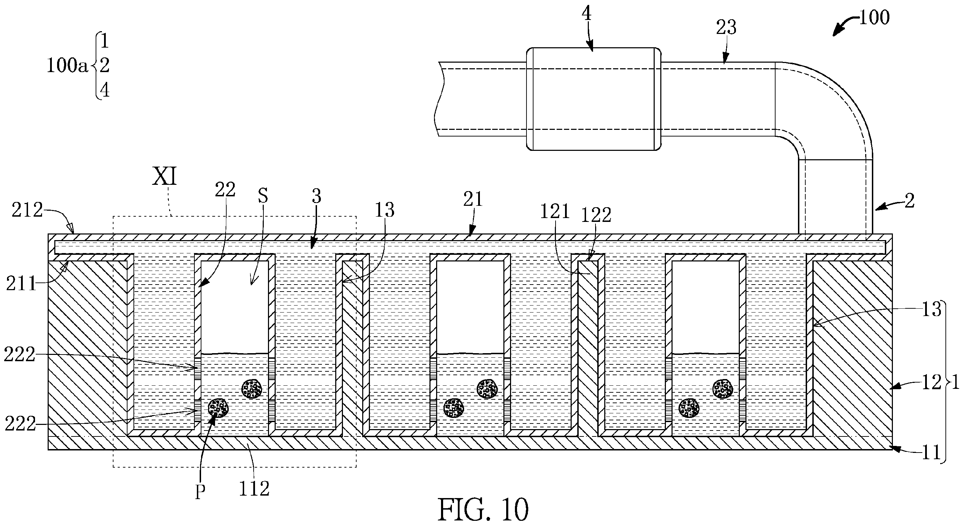

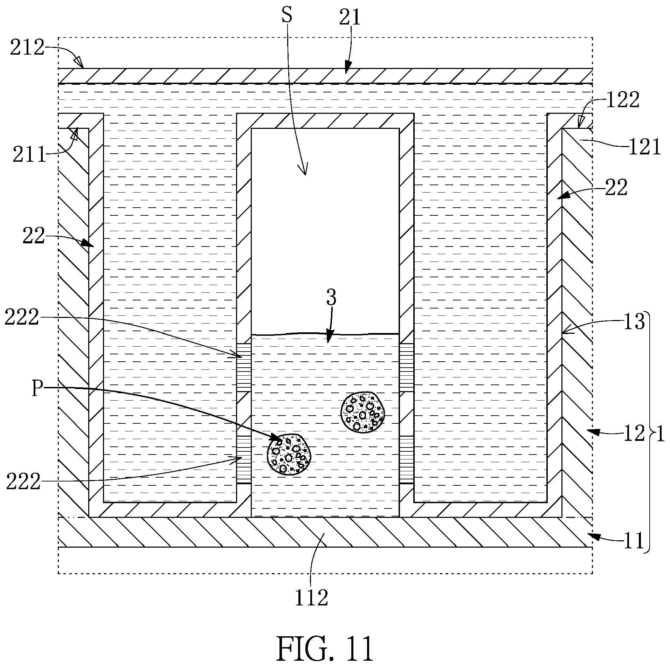

Referring to FIG. 10 and FIG. 11, a third embodiment of the present invention, which is similar to the first embodiment of the present invention, is provided. For the sake of brevity, descriptions of the same components in the first and third embodiments of the present invention will be omitted herein, and the following description only discloses different features between the first and third embodiments.

In the present embodiment, each of the accommodating wells 13 of the 3D culture device 100 has a bottom structure 112 and a surrounding wall 121 that is connected to the bottom structure 112. The bottom structure 112 does not have a filtering function, and the surrounding wall 121 is substantially identical to that of the first embodiment.

Moreover, each of the insertion pipes 22 of the cover 2 can have a plurality of filtering holes 222 formed on a portion thereof (e.g., a side wall) other than a nozzle 221 thereof. Moreover, each of the insertion pipes 22 is able to be in spatial communication with the corresponding culture space S through the filtering holes 222 thereof. In other words, the cover 2 (or the insertion pipes 22) in the present embodiment can be in spatial communication with the culture spaces S only through the filtering holes 222.

Specifically, the filtering holes 222 of each of the insertion pipes 22 are arranged adjacent to the corresponding bottom structure 112 and are immersed in the culture medium 3, and the filtering holes 222 of each of the insertion pipes 22 preferably have an average aperture being less than 1 µm. Accordingly, the driving mechanism 4 is configured to drive the culture medium 3 in the cover 2 so as to allow the culture medium 3 to flow into or out of each of the culture spaces S for controlling the volume of the culture medium 2 in each of the culture spaces S.

In addition, in order to enable the culture medium 3 to smoothly flow through the filtering holes 222 of each of the insertion pipes 22, the nozzle 221 of each of the insertion pipes 22 is preferably closed. For example, the end of each of the insertion pipes 22 abuts against the corresponding bottom structure 112 for enclosing the nozzle 221 thereof; or, each of the insertion pipes 22 is formed to have a closed nozzle 221.

### [Beneficial Effects of the Embodiments]

In conclusion, in any one of the 3D culture device and the biological culture instrument provided by the present embodiment, the structural configuration of the accommodating wells and the cover (e.g., the culture spaces being in spatial communication with the insertion pipes and the communication portion for facilitating filling of the culture medium) enables the volume of the culture medium in each of the accommodating wells to be stably controlled in a liquid-pressure manner, thereby facilitating the culturing of the bioparticles.

Specifically, in any one of the 3D culture device and the biological culture instrument provided by the present embodiment, since the culture spaces is in spatial communication with the insertion pipes and the communication portion for being jointly filled with the culture medium, the culture medium is capable of transmitting biological information generated from the bioparticles respectively located in the culture spaces, thereby allowing a survival rate of the bioparticles P to be effectively increased.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its scope.

## Claims

1. A three-dimensional (3D) culture device (100) for culturing a plurality of bioparticles (P), the 3D culture device (100) **characterized by** comprising:
a plurality of accommodating wells (13) each having a filtering bottom structure (111) and a surrounding wall (121) that is connected to the filtering bottom structure (111), wherein the surrounding wall (121) of each of the accommodating wells (13) defines a culture space (S) therein for accommodating at least one of the bioparticles (P);
a cover (2) including a communication portion (21) and a plurality of insertion pipes (22) that are connected to the communication portion (21), wherein the insertion pipes (22) are in spatial communication with each other through the communication portion (21), and wherein the insertion pipes (22) are respectively inserted into the culture spaces (S) of the accommodating wells (13), and each of the insertion pipes (22) is in spatial communication with the corresponding culture space (S) through the filtering bottom structure (111) of the corresponding accommodating well (13);
a culture medium (3) that is filled in the culture spaces (S) of the accommodating wells (13), the communication portion (21) of the cover (2), and the insertion pipes (22) of the cover (2), so that the bioparticles (P) respectively located in the culture spaces (S) are immersed in the culture medium (3); and
a driving mechanism (4) assembled to the cover (2), wherein the driving mechanism (4) is configured to drive the culture medium (3) in the cover (2) so as to allow the culture medium (3) to flow into or out of each of the culture spaces (S) for controlling volume of the culture medium (3) in each of the culture spaces (S).

2. The 3D culture device (100) according to claim 1, wherein the cover (2) encloses each of the accommodating wells (13), so that the culture space (S) of each of the accommodating wells (13) is only in spatial communication with at least one of the insertion pipes (22) arranged therein.

3. The 3D culture device (100) according to claim 1, wherein the 3D culture device (100) allows the culture medium (3) to flow among the culture spaces (S) through the cover (2) and the filtering bottom structures (111) of the accommodating wells (13), so that the culture medium (3) is capable of transmitting biological information generated from the bioparticles (P) respectively located in the culture spaces (S).

4. The 3D culture device (100) according to claim 1, wherein, in each of the accommodating wells (13), the filtering bottom structure (111) includes a base (1112) and a plurality of upright arms (1111) that are connected to the base (1112) and that are spaced apart from each other, and wherein, in the filtering bottom structure (111) of each of the accommodating wells (13), any two of the upright arms (1111) adjacent to each other have a gap (G) therebetween being smaller than a diameter of any one of the bioparticles (P).

5. The 3D culture device (100) according to claim 4, wherein the gap (G) is less than or equal to 2 µm, and the upright arms (1111) have an average height (H) being within a range from 5 µm to 15 µm.

6. The 3D culture device (100) according to claim 4, further comprising:
a carrier (11) having a plate-like shape and including the filtering bottom structures (111) that are spaced apart from each other; and
a frame (12) that is connected to the carrier (11) to jointly form the accommodating wells (13), wherein the frame (12) includes the surrounding walls (121) that are spaced apart from each other.

7. The 3D culture device (100) according to claim 6, wherein the carrier (11) and the frame (12) are a semiconductor chip, and the driving mechanism (4) is a piezoelectric micro-controller.

8. The 3D culture device (100) according to claim 6, wherein the frame (12) has a top end surface (122) connected to the surrounding walls (121), and the communication portion (21) of the cover (2) abuts against the top end surface (122) to enclose the accommodating wells (13).

9. The 3D culture device (100) according to claim 1, wherein the communication portion (21) has a plate-like shape, and the communication portion (21) has a bottom side (211) and a top side (212) that is opposite to the bottom side (211), where the insertion pipes (22) are connected to the bottom side (211) of the communication portion (21), wherein the cover (2) includes an external pipe (23) that is connected to the top side (212) and that is in spatial communication with each of the insertion pipes (22) through the communication portion (21), and wherein the driving mechanism (4) is assembled to the external pipe (23).

10. The 3D culture device (100) according to claim 1, wherein at least one of the insertion pipes (22) has a plurality of filtering holes (222) and is able to be in spatial communication with the corresponding culture space (S) through the filtering holes (222).

11. The 3D culture device (100) according to claim 10, wherein the filtering holes (222) of the at least one of the insertion pipes (22) have an average aperture being less than 1 µm.

12. The 3D culture device (100) according to claim 10, wherein the filtering holes (222) of the at least one of the insertion pipes (22) are arranged adjacent to the corresponding filtering bottom structure (111) and are immersed in the culture medium (3).

13. A biological culture instrument (100a) for accommodating a culture medium (3) to allow a plurality of bioparticles (P) to be cultured therein, the biological culture instrument (100a) **characterized by** comprising:
a container (1) including a plurality of accommodating wells (13) spaced apart from each other, wherein each of the accommodating wells (13) has a filtering bottom structure (111) and a surrounding wall (121) that is connected to the filtering bottom structure (111), wherein the surrounding wall (121) of each of the accommodating wells (13) defines a culture space (S) therein for accommodating the culture medium (3) and at least one of the bioparticles (P);
a cover (2) including a communication portion (21) and a plurality of insertion pipes (22) that are connected to the communication portion (21), wherein the insertion pipes (22) are in spatial communication with each other through the communication portion (21), and wherein the insertion pipes (22) are respectively inserted into the culture spaces (S) of the accommodating wells (13), and each of the insertion pipes (22) is in spatial communication with the corresponding culture space (S) through the filtering bottom structure (111) of the corresponding accommodating well (13); and
a driving mechanism (4) assembled to the cover (2), wherein the driving mechanism (4) is configured to control volume of the culture medium (3) in each of the culture spaces (S) through the cover (2).

14. The biological culture instrument (100a) according to claim 13, wherein the container (1) includes:
a carrier (11) having a platy shape and including the filtering bottom structures (111) that are spaced apart from each other; and
a frame (12) that is connected to the carrier (11) to jointly form the accommodating wells (13), wherein the frame (12) includes the surrounding walls (121) spaced apart from each other and a top end surface (122) connected to the surrounding walls (121);
wherein the communication portion (21) of the cover (2) abuts against the top end surface (122) to enclose the accommodating wells (13).

15. The biological culture instrument (100a) according to claim 14, wherein, in each of the accommodating wells (13), the filtering bottom structure (111) includes a plurality of upright arms (1111) spaced apart from each other, and wherein, in the filtering bottom structure (111) of each of the accommodating wells (13), any two of the upright arms (1111) adjacent to each other have a gap (G) therebetween being smaller than a diameter of any one of the bioparticles (P).

16. The biological culture instrument (100a) according to claim 15, wherein the gap (G) is less than or equal to 2 µm, and the upright arms (1111) have an average height (H) being within a range from 5 µm to 15 µm.

17. The biological culture instrument (100a) according to claim 13, wherein the communication portion (21) has a plate-like shape, and the communication portion (21) has a bottom side (211) and a top side (212) that is opposite to the bottom side (211), wherein the insertion pipes (22) are connected to the bottom side (211) of the communication portion (21), wherein the cover (2) includes an external pipe (23) that is connected to the top side (212) and that is in spatial communication with each of the insertion pipes (22) through the communication portion (21), and wherein the driving mechanism (4) is assembled to the external pipe (23).

18. The biological culture instrument (100a) according to claim 13, wherein at least one of the insertion pipes (22) has a plurality of filtering holes (222) and is able to be in spatial communication with the corresponding culture space (S) through the filtering holes (222), and wherein the filtering holes (222) of the at least one of the insertion pipes (22) have an average aperture being less than 1 µm.

19. A three-dimensional (3D) culture device for culturing a plurality of bioparticles (P), the 3D culture device (100) **characterized by** comprising:
a container (1) including a plurality of accommodating wells (13) spaced apart from each other, wherein each of the accommodating wells (13) has a bottom structure (112) and a surrounding wall (121) that is connected to the bottom structure (112), wherein the surrounding wall (121) of each of the accommodating wells (13) defines a culture space (S) therein for accommodating at least one of the bioparticles (P);
a cover (2) including a communication portion (21) and a plurality of insertion pipes (22) that are connected to the communication portion (21), wherein the insertion pipes (22) are in spatial communication with each other through the communication portion (21), and wherein each of the insertion pipes (22) has a plurality of filtering holes (222), the insertion pipes (22) are respectively inserted into the culture spaces (S) of the accommodating wells (13), and each of the insertion pipes (22) is in spatial communication with the corresponding culture space (S) through the filtering holes (222) thereof;
a culture medium (3) that is filled in the culture spaces (S) of the accommodating wells (13), so that the bioparticles (P) respectively located in the culture spaces (S) are immersed in the culture medium (3); and
a driving mechanism (4) assembled to the cover (2), wherein the driving mechanism (4) is configured to drive the culture medium (3) in the cover (2) so as to allow the culture medium (3) to flow into or out of each of the culture spaces (S) for controlling volume of the culture medium (3) in each of the culture spaces (S).

20. The 3D culture device (100) according to claim 19, wherein the filtering holes (222) of each of the insertion pipes (22) are arranged adjacent to the corresponding bottom structure (112) and are immersed in the culture medium (3), and the filtering holes (222) of the each of the insertion pipes (22) have an average aperture being less than 1 µm.
